(19) Europäisches Patentamt
European Patent Office
Office européen des brevets

(11) Veröffentlichungsnummer: **0 050 790**
**B1**

(12) # EUROPÄISCHE PATENTSCHRIFT

(45) Veröffentlichungstag der Patentschrift: **19.12.84**

(51) Int. Cl.³: **C 07 D241/44**

(21) Anmeldenummer: **81108262.7**

(22) Anmeldetag: **13.10.81**

(54) **Herstellung von in alpha-Stellung chlorierten Stickstoff-Heterocyclen.**

(30) Priorität: **23.10.80 DE 3040000**

(43) Veröffentlichungstag der Anmeldung: **05.05.82 Patentblatt 82/18**

(45) Bekanntmachung des Hinweises auf die Patenterteilung: **19.12.84 Patentblatt 84/51**

(84) Benannte Vertragsstaaten: **CH DE FR LI**

(56) Entgegenhaltungen:
**DE-A- 1 620 118**
**DE-A- 1 695 532**
**DE-B- 1 178 052**
**DE-C- 910 893**

(73) Patentinhaber: **BAYER AG**
**Konzernverwaltung RP Patentabteilung**
**D-5090 Leverkusen 1 Bayerwerk (DE)**

(72) Erfinder: **Blank, Heinz-Ulrich, Dr.**
**Am Geusfelde 35**
**D-5068 Odenthal (DE)**
Erfinder: **Langenfeld, Norbert, Dr.**
**Hofstrasse 53**
**D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 050 790 B1**

Jouve, 18, rue St-Denis, 75001 Paris, France

**Beschreibung**

Die vorliegende Erfindung betrifft ein Verfahren zur Herstellung von Stickstoff-Heterocyclen der Reihe Chinolin, Isochinolin, Chinoxalin, Chinazolin und Phthalazin, die in α-Stellung zum Ring-Stickstoff mindestens ein Chloratom enthalten, durch Umsetzung von entsprechenden Stickstoff-Heterocyclen, die in α-Stellung zum Ring-Stickstoff mindestens eine Hydroxylgruppe enthalten, mit Thionylchlorid in Gegenwart von substituierten Formamiden.

Aus DE-C 910 893 ist es bekannt, daß man 1,3,5-Triazine, die im heterocyclischen Ring Chloratome tragen, durch Umsetzen der entsprechenden Hydroxyverbindungen mit Thionylchlorid unter Zusatz von Phosphorpentachlorid in einem inerten organischen Lösungsmittel herstellen kann. Dieses Verfahren erfordert den Einsatz sehr großer Lösungsmittelmengen (etwa 20 Teile auf 1 Teil Ausgangsmaterial), was zu niedrigen Raum/Zeit-Ausbeuten und zu einer aufwendigen destillativen Abtrennung des Lösungsmittels führt.

Nach dem Verfahren von DE-B 11 78 052 werden heterocyclische Verbindungen, die mindestens ein zu einem Ring-Stickstoffatom α-ständiges Chloratom enthalten, hergestellt, indem man zu dem in überschüssigem Thionylchlorid suspendierten Hydroxy-Stickstoff-Heterocyclus nach und nach N,N-dialkylsubstituiertes Formamid, dessen Alkylgruppen bis zu je 4 Kohlenstoffatome aufweisen, zugibt. Nun ist aber bekannt, daß aus niederen N,N-disubstituierten Formamiden, beispielsweise aus Dimethylformamid, mit Thionylchlorid, N,N-disubstituierte Carbamoylchloride entstehen (Isw. Akad. Nauk USSR, Chem. Ser. 1971 (3), 513-519 ; zitiert nach C.A. *75,* 48340m), die sich im Tierversuch als krebserregend erwiesen haben (J. Nat. Cancer Inst. *48* (5), 1539-1541 (1972) ; zitiert nach C.A. *77,* 97540b). Diese Carbamoylchloride sind sowohl im abdestillierten überschüssigen Thionylchlorid als auch im Gasraum über dem Reaktionsprodukt nachweisbar.

Aus DE-A 1 695 532 ist im Zuge der Herstellung eines Zwischenproduktes die Umsetzung von 2,3-Dihydroxy-7-chlorchinoxalin-6-carbonsäure mit Phosphoroxychlorid zu 6-Chlorcarbonyl-2,3,7-trichlorchinoxalin bekannt. (Siehe dazu DE-A 1 695 532 Beispiel 4, Stufe B).

DE-A 1 620 118 betrifft ein Verfahren zur Herstellung von Chloriden von Chlorchinoxalincarbonsäuren, wobei Hydroxychinoxalincarbonsäuren mit Phosgen in Gegenwart geringer Mengen eines Carbonsäureamids umgesetzt werden.

Es wurde nun ein neues Verfahren zur Herstellung von Stickstoff-Heterocyclen der Reihe Chinolin, Isochinolin, Chinoxalin, Chinazolin und Phthalazin, die in α-Stellung zum Ring-Stickstoff mindestens ein Chloratom enthalten durch Umsetzung von entsprechenden Stickstoff-Heterocyclen, die in α-Stellung zum Ring-Stickstoff mindestens eine Hydroxylgruppe enthalten mit Thionylchlorid in Gegenwart von substituierten Formamiden gefunden.

Das Verfahren ist dadurch gekennzeichnet, daß als substituierte Formamide solche der Formel

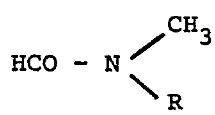

eingesetzt werden, in der

R = Alkyl mit 10-26 C-Atomen, Phenyl, Naphthyl, Anthryl oder Biphenyl oder solches Alkyl, in dem ein oder mehrere C-Atome durch $—NCH_3—$ oder —NCHO— ersetzt sind,

und daß 0,001 bis 1 Mol substituiertes Formamid pro umzusetzende Hydroxylgruppe eingesetzt und die Umsetzung bei 50 bis 200 °C durchgeführt wird.

Als Alkyl mit 10-26 C-Atomen seien genannt : Decyl, Dodecyl, Palmityl, Stearyl, Eikosanyl, Hexaneikosanyl.

Die Substituenten R, insbesondere die aromatischen Teile solcher Substituenten, können ihrerseits durch weitere inerte Substituenten, wie Fluor, Chlor, Brom, Jod, Alkoxy, Aryloxy oder Nitro substituiert sein.

Beispiele für erfindungsgemäß einsetzbare substituierte Formamide sind : N-Methyl-palmitylformamid, N-Methyl-stearylformamid, N-Methyl-eicosanylformamid, N-Methyl-docosanylformamid. Als eines der bevorzugt einzusetzenden Formamide sei N-Methyl-N-stearylformamid genannt. Die Herstellung solcher N,N-dialkylierten Formamide erfolgt nach an sich bekannten Methoden, beispielsweise der Formylierung von Aminen mit Ameisensäure oder ihren Salzen, mit Ameisensäure- und Essigsäureanhydrid, mit Ameisensäureestern, mit Formamid, mit Formylfluorid, mit Chloral oder mit Kohlenmonoxid (Houben-Weyl, Methoden der Organischen Chemie, Band XI/2, Seite 27 bis 30, Georg Thieme-Verlag, Stuttgart, 1958).

Beim erfindungsgemäßen Verfahren werden vorzugsweise 0,005-0,5 Mol substituiertes Formamid pro umzusetzende Hydroxylgruppe eingesetzt.

Insbesondere werden 0,05-0,1 Mol substituiertes Formamid pro umzusetzende Hydroxylgruppe eingesetzt.

Das als Ausgangsverbindung einsetzbare N-Methylformanilid ist nach Houben-Weyl, Methoden der

Organischen Chemie, Band XI/2 S. 28 beispielsweise aus N-Methylanilin durch Erhitzen mit Ameisensäure in Benzol zugänglich. In analoger Weise sind auch die anderen substituierten Formamide durch Formylierung herstellbar.

Als Ausgangsstoffe für das erfindungsgemäße Verfahren sind heterocyclische Verbindungen der Reihe Chinolin, Isochinolin, Chinoxalin, Chinazolin und Phthalazin, die α-ständig zu einem Ringstickstoffatom mindestens eine Hydroxygruppe tragen, oder deren tautomere Formen, geeignet. Die Heterocyclen können auch mehrere α-ständige Hydroxygruppen zu einem Ringstickstoffatom tragen. Diese heterocyclischen Ringsysteme können außer den genannten Hydroxysubstituenten noch ein oder mehrere der folgenden Substituenten enthalten, die gegebenenfalls unter den erfindungsgemäßen Bedingungen ebenfalls in die Chloride umgewandelt werden : Beispielsweise genannt seinen die Carbonsäuregruppe oder die Sulfonsäuregruppe.

Selbstverständlich können die erfindungsgemäß einsetzbaren heterocyclischen Verbindungen Substituenten tragen, die unter den Chlorierungsbedingungen nicht verändert werden, wie niederes Alkyl, Halogene, wie Fluor, Chlor, Brom, niederes Alkoxy, Cyano, Nitro und niederes Halogenalkyl.

Ausgangsstoffe für das erfindungsgemäße Verfahren leiten sich in bevorzugter Weise vom Chinoxalin ab.

Beispiele für Einzelverbindungen, die erfindungsgemäß als Ausgangsstoffe eingesetzt werden können sind : 2-Hydroxychinolin, 2,4-Dihydroxychinazolin, 6-Chlor-2,4-dihydroxychinazolin, 2,3-Dihydroxy-chinoxalin, 6-Chlor-2,3-dihydroxychinoxalin, 6-Methyl-2,3-dihydroxy-chinoxalin, 6,7-Dimethoxy-2,3-dihydroxychinoxalin, 2,3-Dihydroxychinoxalin-6-carbonsäure, 2,3-Dihydroxy-5-methylchinoxalin, 2,3-Dihydroxy-6,7-dimethyl-chinoxalin und 2,3-Dihydroxy-chinoxalin-5-carbonsäure.

Ein Lösungsmittel ist für das erfindungsgemäße Verfahren nicht erforderlich. Es ist aber durchaus möglich, die Reaktion in einem gegenüber Thionylchlorid inerten Reaktionsmedium durchzuführen, beispielsweise in Benzol, Toluol, Xylol, Chlorbenzol, Dichlorbenzol, Trichlorbenzol, Isododecan, Trichlorethan oder Tetrachlorethan.

Ebenso kann überschüssiges Thionylchlorid als Reaktionsmedium dienen.

Zur Durchführung der erfindungsgemäßen Reaktion ist das stöchiometrische Verhältnis von Thionylchlorid zu dem eingesetzten Stickstoff-Heterocyclus ohne besondere Bedeutung. Zur Vervollständigung der Reaktion ist es jedoch sinnvoll, mindestens ein Mol Thionylchlorid je umzusetzende Hydroxylgruppe zu verwenden. Vorteilhafte Ergebnisse werden mit überschüssigem Thionylchlorid erzielt. Beispielsweise sei eine Menge von 1 bis 5 Mol, bevorzugt 2 bis 4 Mol, Thionylchlorid je umzusetzende Hydroxylgruppe genannt.

Die Menge des erfindungsgemäß eingesetzten substituierten Formamids liegt zwischen 0,001 und 1 Mol pro umzusetzende OH-Gruppe. Bevorzugt ist der Bereich zwischen 0,005 und 0,5 Mol, besonders bevorzugt ist der Bereich zwischen 0,05 und 0,1 Mol, bezogen auf jede OH-Gruppe. Die Reaktionskomponenten und das erfindungsgemäß einzusetzende substituierte Formamid können in beliebiger Reihenfolge zusammengegeben werden.

Das erfindungsgemäße Verfahren wird bei einer Temperatur von 50 bis 200 °C, bevorzugt 60 bis 150 °C, besonders bevorzugt 70 bis 100 °C, durchgeführt. Wird die Reaktion mit einem Überschuß an Thionylchlorid durchgeführt, so ist es in vielen Fällen vorteilhaft, bei der Siedetemperatur des Thionylchlorids zu arbeiten.

Das erfindungsgemäße Verfahren wird im allgemeinen bei Normaldruck durchgeführt. Es ist aber auch möglich, bei vermindertem oder erhöhtem Druck zu arbeiten. So kann es beispielsweise vorteilhaft sein, durch Erhöhung des Druckes die Siedetemperatur des Thionylchlorids und damit die maximale Reaktionstemperatur zu erhöhen, was zur Beschleunigung der Reaktion beitragen kann.

Die Aufarbeitung des Reaktionsansatzes erfolgt im allgemeinen durch Abdestillieren des überschüssigen Thionylchlorids. Das im Rückstand verbleibende Reaktionsprodukt ist für viele Anwendungen schon von ausreichender Reinheit. Eine weitere Reinigung des Produktes kann durch bekannte Verfahren erfolgen, beispielsweise durch Destillation, Sublimation, Extraktion oder Umkristallisation. Das erfindungsgemäße Verfahren kann sowohl kontinuierlich als auch diskontinuierlich durchgeführt werden.

Das erfindungsgemäße Verfahren weist gegenüber Verfahren in Gegenwart von Phosphorpentachlorid den Vorteil der leichteren Handhabbarkeit des Thionylchlorids auf. Ein weiterer Vorteil besteht darin, daß als Nebenprodukte nur leicht flüchtiges Schwefeldioxid und Chlorwasserstoff entstehen, während die Reaktionsprodukte des Phosphorpentachlorids nur schwer vom Produktgemisch abzutrennen sind. Bei Verwendung von Thionylchlorid wird weiterhin die Bildung von unerwünschten, z. B. weiterchlorierten Nebenprodukten unterdrückt. N,N-Dialkylcarbamoylchloride, die bei der erfindungsgemäßen Reaktion entstehen, sind sehr schwer flüchtig. Während beispielsweise Carbamoylchloride, die aus den Formamiden mit kurzen Alkylresten entstehen, sowohl im Gasraum über dem Reaktionsgemisch bzw. dem Reaktionsprodukt als auch im überschüssigen Thionylchlorid, das durch Destillation zurückgewonnen wird, auftreten, befinden sich Carbamoylchloride, die aus den erfindungsgemäß einsetzbaren substituierten Formamiden entstehen können, nur im Produkt oder nur in untergeordnetem Maße im Gasraum über den Produkten oder in wiedergewonnenen Thionylchlorid.

Es ist überraschend, daß auch substituierte Formamide mit längeren Alkylketten und mit anderen als Alkylsubstituenten im beschriebenen Verfahren katalytisch wirksam sind.

In Helv. Chim. Acta, *42* (1959) 1653-1658, wo die Umsetzung von Carbon- und Sulfonsäure mit Thionylchlorid beschrieben sind, benutzen die Autoren Dimethylformamid als Katalysator, da die Wirksamkeit anderer Formamidderivate als geringer angesehen wurde.

Beispiel 1 (Vergleichsbeispiel)

113,2 g 2,3-Dihydroxychinoxalin-6-carbonsäure mit einem Gehalt von 91 % werden mit 393 g Thionylchlorid und 3,0 g N,N-Dimethylformamid in einem 0,5-Ltr.-Dreihalskolben, der mit Rührer, Innenthermometer und Rückflußkühler mit Blasenzähler und Gasableitung versehen ist, zum Sieden erhitzt. Es setzt eine heftige Gasentwicklung ein. Es wird unter Rückfluß erhitzt, bis die ursprüngliche Suspension in eine Lösung übergegangen und die Gasentwicklung beendet ist.

Überschüssiges Thionylchlorid wird bei 20 mbar bis zu einer Sumpftemperatur von 120 °C abdestilliert. Es werden 100 g Thionylchlorid wiedergewonnen. Der flüssige Rückstand wird vorsichtig auf ein Blech gegossen und nach dem Erkalten im Mörser pulverisiert. Es werden 135 g 2,3-Dichlorchinoxalin-6-carbonsäurechlorid isoliert. Der durch Sublimation bestimmte Gehalt beträgt 96 %.

Die Ausbeute beträgt 99 % der theoretischen Ausbeute. Gehalt an N,N-Dimethylcarbamoylchlorid :

| | |
|---|---|
| a) im Gasraum über dem 140 °C heißen Produkt | 55 ppm |
| b) im Thionylchlorid | 0,5 % |

Beispiel 2

227 g 2,3-Dihydroxychinoxalin-6-carbonsäure mit einem Gehalt von 91 % werden in einem 1 Ltr.-Dreihalskolben, der mit Rührer, Innenthermometer und Rückflußkühler mit aufgesetztem Blasenzähler und Gasableitung versehen ist, vorgelegt. Dann werden bei Raumtemperatur 785 g Thionylchlorid und 18,6 g N-Methyl-stearylformamid zugegeben. Der Ansatz wird zum Sieden erhitzt, wobei eine heftige Gasentwicklung einsetzt. Die Siedetemperatur steigt während der Reaktion von ca. 55 °C auf 79 °C. Nach etwa 5 Stunden geht die Suspension in eine Lösung über, und nach weiteren 1-2 Stunden ist die Reaktion beendet, was am Ende der Gasentwicklung zu erkennen ist. Überschüssiges Thionylchlorid wird zunächst bei Normaldruck, dann bei 20 mbar bis zu einer Sumpftemperatur von 120 °C abdestilliert. Es werden 130 g Thionylchlorid wiedergewonnen.

Der flüssige Rückstand wird auf ein Blech gegossen und nach dem Erkalten im Mörser pulverisiert. Es werden 276 g 2,3-Dichlorchinoxalin-6-carbonsäurechlorid isoliert. Das Produkt hat einen durch Sublimation bestimmten Gehalt von 94 %. Die Ausbeute beträgt 99 % der theoretischen Ausbeute.

Gehalt an N-Methyl-N-stearylcarbamoylchlorid :

| | |
|---|---|
| a) im Gasraum über dem 140 °C heißen Produkt | nicht nachweisbar |
| b) im Thionylchlorid | nicht nachweisbar. |

Beispiel 3

113,2 g 2,3-Dihydroxychinoxalin-6-carbonsäure mit einem Gehalt von 91 % werden in einem 0,5-Ltr.-Dreihalskolben mit 393 g Thionylchlorid in Gegenwart von 5,4 g N-Methyl-phenyl-formanilid analog Beispiel 2 umgesetzt und aufgearbeitet.

Es werden 103 g Thionylchlorid wiedergewonnen.

Die Aufarbeitung ergibt 136,4 g 2,3-Dichlorchinoxalin-6-carbonsäurechlorid mit einem durch Sublimation bestimmten Gehalt von 95 %. Die Ausbeute beträgt 99 % der theoretischen Ausbeute.

Gehalt an N-Methyl-N-phenylcarbamoylchlorid :

| | |
|---|---|
| a) im Gasraum über dem 140 °C heißen Produkt | nicht nachweisbar |
| b) im Thionylchlorid | 100 ppm. |

Beispiel 4

88 g 2,3-Dihydroxy-6-methylchinoxalin werden in einem 0,5-Ltr.-Dreihalskolben mit 333 g Thionylchlorid in Gegenwart von 10 g N-Methyl-stearylformamid analog Beispiel 2 umgesetzt und aufgearbeitet.

Es werden 170 g Thionylchlorid wiedergewonnen.

Die Aufarbeitung ergibt 115 g 2,3-Dichlor-6-methylchinoxalin mit einem Gehalt von 92 %. Die Ausbeute beträgt 99 % der theoretischen Ausbeute.

**Ansprüche**

1. Verfahren zur Herstellung von Stickstoff-Heterocyclen der Reihe Chinolin, Isochinolin, Chinoxalin, Chinazolin und Phthalazin, die in α-Stellung zum Ring-Stickstoff mindestens ein Chloratom enthalten,

durch Umsetzung von entsprechenden Stickstoff-Heterocyclen, die in α-Stellung zum Ring-Stickstoff mindestens eine Hydroxylgruppe enthalten, mit Thionylchlorid in Gegenwart von substituierten Formamiden, dadurch gekennzeichnet, daß als substituierte Formamide solche der Formel

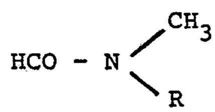

eingesetzt werden, in der

R = Alkyl mit 10-26 C-Atomen, Phenyl, Naphthyl, Anthryl oder Biphenyl oder solches Alkyl, in dem ein oder mehrere C-Atome durch $—NCH_3—$ oder —NCHO— ersetzt sind,

und, daß 0,001 bis 1 Mol substituiertes Formamid pro umzusetzende Hydroxylgruppe eingesetzt und die Umsetzung bei 50 bis 200 °C durchgeführt wird.

2. Verfahren nach Anspruch 1, dadurch gekennzeichnet, daß 0,005-0,5 Mol substituiertes Formamid pro umzusetzende Hydroxylgruppe eingesetzt wird.

3. Verfahren nach Anspruch 1 bis 2, dadurch gekennzeichnet, daß 0,05-0,1 Mol substituiertes Formamid pro umzusetzende Hydroxylgruppe eingesetzt wird.

4. Verfahren nach Anspruch 1 bis 3, dadurch gekennzeichnet, daß bei 60 bis 150 °C gearbeitet wird.

**Claims**

1. Process for the preparation of nitrogen-heterocyclic compounds from the series comprising quinoline, isoquinoline, quinoxaline, quinazoline and phthalazine, which contain at least one chlorine atom in the α-position to the ring nitrogen, by reacting corresponding nitrogen-heterocyclic compounds which contain at least one hydroxyl group in the α-position to the ring nitrogen with thionyl chloride in the presence of substituted formamides, characterised in that the substituted formamides used are those of the formula

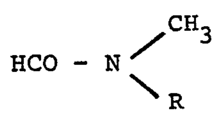

in which

R = alkyl with 10-26 C atoms, phenyl, naphthyl, anthryl or biphenyl or such an alkyl in which one or more C atoms are replaced by $—NCH_3—$ or —NCHO—,

and in that 0.001 to 1 mol of substituted formamide is used per hydroxyl group to be reacted and the reaction is carried out at 50 to 200 °C.

2. Process according to Claim 1, characterised in that 0.005-0.5 mol of substituted formamide is used per hydroxyl group to be reacted.

3. Process according to Claim 1 to 2, characterised in that 0.05-0.1 mol of substituted formamide is used per hydroxyl group to be reacted.

4. Process according to Claim 1 to 3, characterised in that it is carried out at 60 to 150 °C.

**Revendications**

1. Procédé de production d'hétérocycles azotés de la série de la quinoléine, de l'isoquinoléine, de la quinoxaline, de la quinazoline et de la phtalazine, qui portent en position α par rapport à l'azote du noyau au moins un atome de chlore, par réaction d'hétérocycles azotés correspondants qui portent en position α par rapport à l'azote du noyau au moins un groupe hydroxyle, avec le chlorure de thionyle en présence de formamides substitués, caractérisé en ce qu'on utilise comme formamides substitués des composés de formule

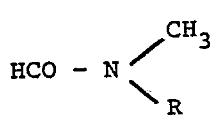

dans laquelle

R est un groupe alkyle ayant 10 à 26 atomes de carbone, phényle, naphtyle, anthryle ou biphényle ou un groupe alkyle dans lequel un ou plusieurs atomes de carbone sont remplacés par un groupe $—NCH_3—$ ou —NCHO—,

et en ce qu'on utilise 0,001 à 1 mole de formamide substitué par groupe hydroxyle devant réagir et on conduit la réaction à 50-200 °C.

2. Procédé suivant la revendication 1, caractérisé en ce qu'on utilise 0,005 à 0,5 mole de formamide substitué par groupe hydroxyle devant réagir.

3. Procédé suivant les revendications 1 et 2, caractérisé en ce qu'on utilise 0,05 à 0,1 mole de formamide substitué par groupe hydroxyle devant réagir.

4. Procédé suivant les revendications 1 à 3, caractérisé en ce qu'on opère à une température de 60 à 150 °C.